(19) Europäisches Patentamt
European Patent Office
Office européen des brevets

(11) Numéro de publication : **0 015 171**
**B1**

(12) # FASCICULE DE BREVET EUROPÉEN

(45) Date de publication du fascicule du brevet : **28.04.82**

(21) Numéro de dépôt : **80400097.4**

(22) Date de dépôt : **22.01.80**

(51) Int. Cl.³ : **C 07 D471/04**, C 07 D487/04, A 61 K 31/415, A 61 K 31/435, A 61 K 31/505, A 61 K 31/55// C07D207/22, C07D211/72 ,(C07D471/04, 235/00, 221/00),(C07D487/04, 235/00, 209/00),(C07D487/04, 239/00, 209/00),(C07D487/04, 243/00, 209/00)

(54) **Dérivés condensés de pyrrolidine ou de pipéridine, leur procédé de préparation et composition les contenant.**

(30) Priorité : **20.02.79 FR 7904266**

(43) Date de publication de la demande : **03.09.80 (Bulletin 80/18)**

(45) Mention de la délivrance du brevet : **28.04.82 Bulletin 82/17**

(84) Etats contractants désignés : **BE CH DE GB IT NL SE**

(56) Documents cités :
**FR - A - 2 299 029**
**FR - M - 4 530**
**FR - M - 7 813**

(73) Titulaire : **LABORATOIRES JACQUES LOGEAIS Société dite:**
**71, Avenue du Général de Gaulle**
**F-92130 Issy-Les-Moulineaux (FR)**

(72) Inventeur : **Maillard, Jacques**
**82 bis, Avenue de Paris**
**F-78000 Versailles (FR)**
Inventeur : **Vo Van, Tri**
**5 bis, Rue du Centre**
**F-91430 Igny (FR)**
Inventeur : **Legeai, Jacky Marcel**
**3, Résidence Marceau**
**F-91120 Palaiseau (FR)**

(74) Mandataire : **Bressand, Georges et al**
**c/o CABINET LAVOIX 2 Place d'Estienne d'Orves**
**F-75441 Paris Cedex 09 (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Imprimerie Jouve, 18, rue St-Denis, 75001 Paris, France

EP 0 015 171 B1

Dérivés condensés de pyrrolidine ou de pipéridine, leur procédé de préparation et composition les contenant

La présente invention concerne de nouveaux dérivés condensés de pyrrolidine ou de pipéridine, leur procédé de préparation et leurs applications en thérapeutique, notamment comme anti-dépresseurs.

Le brevet FRM 4530 décrit des pipéridino [1,2-a] imidazolinones qui peuvent comporter comme substituant sur la partie pipéridine uniquement un radical alkyle et qui sont donc dépourvus sur cette partie pipéridine de substituant de type aromatique. Ces composés sont essentiellement des inhibiteurs de la mono-amine-oxydase. Ils ont en outre une activité sur le système nerveux central mais qui peut être soit dépressive, soit stimulante ou encore mixte selon les composés.

Le brevet FRM 7813 décrit des dérivés de l'homopyrimidazole qui ne comporte pas non plus de substituant de type aromatique sur le noyau monoazoté. Ces composés sont décrits essentiellement comme des analgésiques.

La présente invention a pour objet des composés de formule

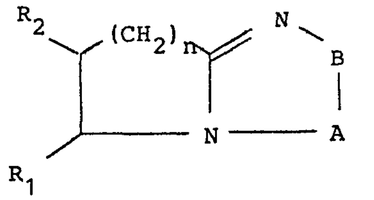

(I)

dans laquelle

*n* est égal à 1 ou 2,

$R_1$ représente un atome d'hydrogène et $R_2$ représente un groupe phényle ou un groupe benzofuryle-2, ou bien $R_2$ représente un atome d'hydrogène et $R_1$ représente un groupe phényle ou un groupe benzyle, et

le groupement —A—B— représente un groupe de formule

$$-CO-\underset{R}{\underset{|}{CH}}- \quad , \quad -CO-\underset{R}{\underset{|}{CH}}-CH_2- \text{ ou } -CO-(CH_2)_3-$$

R représentant un atome d'hydrogène, un radical alcoyle en $C_1$ à $C_6$, un groupe phényle, un groupe benzyle, un groupe méthylthioéthyle ou un groupe de formule $-(CH_2)_m-COOR'$, *m* étant égal à 0, 1 ou 2 et R' étant un radical alcoyle en $C_1$ à $C_6$, ainsi que leurs sels avec des acides pharmacologiquement acceptables.

Les sels peuvent être notamment ceux formés avec les acides chlorhydrique, sulfurique, phosphorique, méthane-sulfonique, maléique, succinique, pamoïque, acétique, fumarique, lactique, aspartique et citrique.

Les composés de formule I peuvent être préparés par réaction d'un composé de formule

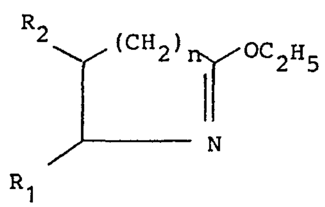

(II)

dans laquelle *n*, $R_1$ et $R_2$ ont la signification donnée ci-dessus,
avec une amine de formule

$$R''O-A-B-NH_2 \qquad (III)$$

dans laquelle A et B ont la signification donnée ci-dessus et R" représente un atome d'hydrogène ou un radical alcoyle en $C_1$ à $C_6$,
obtenant ainsi un composé de formule

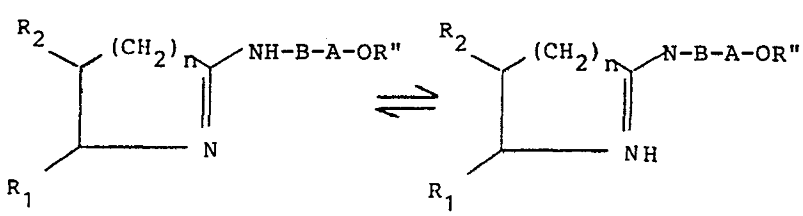

(IV)

et cyclisation du composé ainsi obtenu de formule IV en composé de formule I.

L'amine de formule III peut être utilisée sous forme de base ou de sel tel que le chlorhydrate.

La réaction du composé de formule II avec l'amine de formule III peut être effectuée par chauffage à l'ébullition dans un solvant tel que l'éthanol ou l'isopropanol.

La cyclisation du composé de formule IV peut notamment être effectuée par chauffage de ce composé sous forme de base après évaporation du solvant, ce chauffage étant suivi d'une distillation sous pression réduite.

Dans certains cas, cette cyclisation s'effectue spontanément à la température ambiante.

On peut également chauffer à l'ébullition le composé de formule IV dans un milieu acide aqueux ou alcoolique. De ce fait, l'isolement intermédiaire du composé de formule IV n'est pas indispensable et il est possible d'obtenir directement le composé de formule I par chauffage du composé de formule II avec le composé de formule III sous forme de sel.

Les exemples suivants illustrent la présente invention.

## Exemple 1

Composé (I) $-A-B- = -CO-\underset{\substack{| \\ CH_3}}{CH}-$, n = 1, $R_1 = H$, $R_2 = C_6H_5$

a) Une solution de 9,45 g (0,05 mole) d'éthoxy-2 phényl-4Δl-pyrroline (II, n = 1, $R_1 = H$, $R_2 = C_6H_5$) et 7,7 g de chlorhydrate de DL-alaninate d'éthyle (III, $-A-B- = -CO-\underset{\substack{| \\ CH_3}}{CH}-$, $R'' = C_2H_5$) dans 100 ml d'éthanol, est portée à reflux pendant 24 h. Après évaporation à sec et trituration du résidu avec de l'acétate d'éthyle, on obtient 12 g du produit intermédiaire IV (n = 1, $-A-B- = -CO-\underset{\substack{| \\ CH_3}}{CH}-$ ; $R_1 = H$ ; $R_2 = C_6H_5$ ; $R'' = C_2H_5$) sous forme de chlorhydrate cristallisé. $F_{inst} = 139$ °C - Rendt : 81 %.

b) 11,6 g du dérivé (IV) précédent sont dissous dans du chloroforme et transformés en base par agitation avec une solution saturée de $NaHCO_3$ en excès. Après évaporation du solvant organique, le résidu est cyclisé en base (I) par chauffage, puis distillé lentement sous pression réduite – $eb_{0,25} = 154$ °C. Le produit (I) cristallise par refroidissement. $F_{inst} = 65\text{-}70$ °C - Rendt : 65 %.

c) Le même produit (I) peut être préparé à partir d'éthoxy-2 phényl-4Δl-pyrroline et de DL-alanine (III, $R'' = H$), à ébullition à reflux dans l'éthanol pendant 24 h. Après évaporation et addition d'éther au résidu, le dérivé intermédiaire (IV, $R'' = H$) est obtenu sous forme d'un solide. $F_{inst} = 231$ °C - Rendt : 74 %.

La cyclisation thermique du dérivé (IV), vers 200 °C sous pression réduite, conduit au produit (I) qui distille et cristallise par refroidissement.

## Exemple 2

Composé (I) $-A-B- = -CO-\underset{\substack{| \\ CH \\ / \quad \backslash \\ CH_3 \quad CH_3}}{CH}-$ ; n = 1 ; $R_1 = H$ ; $R_2 = C_6H_5$.

Il est préparé comme le composé (I) de l'exemple 1, à partir de DL-valine (III, $-A-B- = -CO-\underset{\substack{| \\ iC_3H_7}}{CH}-$ ; $R'' = H$) et d'éthoxy-2 phényl-4Δl-pyrroline (II, n = 1 ; $R_1 = H$ ; $R_2 = C_6H_5$).

Le dérivé intermédiaire (IV) est recristallisé dans l'acétate d'éthyle, puis l'isopropanol. $F_{inst} = 275\text{-}280$ °C - Rendt : 96 %.

Le dérivé (IV) est cyclisé en base (I) par chauffage progressif à 250 °C sous 0,1 mm, puis distillation :

$$eb_{0,2} = 152\text{-}156 \text{ °C}.$$

Le produit cristallisé par refroidissement.
$F_{inst} = 58$ °C - Rendt : 73 %.

## Exemple 3

Composé (I) $-A-B- = -CO-\underset{\substack{| \\ C_6H_5}}{CH}-$ ; n = 1 ; $R_1 = H$ ; $R_2 = C_6H_5$.

Il est préparé comme le composé (I) de l'exemple 1, à partir de chlorhydrate de DL-phényl-2 amino-2

acétate d'éthyle (III, —A—B— = —CO—CH ; R″ = $C_2H_5$) et d'éthoxy-2 phényl-4Δl-pyrroline (II, n = 1 ;
|
$C_6H_5$

$R_1$ = H ; $R_2$ = $C_6H_5$). Le produit intermédiaire obtenu sous forme de chlorhydrate par évaporation du solvant, est cristallisé par trituration avec de l'acétate d'éthyle. $F_{inst}$ = 206 °C - Rendt : 82 %.

Le chlorhydrate du dérivé (IV) précédent est transformé par l'ammoniaque en solution éthanolique en base qui se cyclise spontanément en 48 h. La base (I) cristallise dans l'éthanol. $F_{inst}$ = 194 °C - Rendt : 43 %.

## Exemple 4

Composé (I) —A—B— = —CO—CH— ; n = 1 ; $R_1$ = H ; $R_2$ = $C_6H_5$.
|
$CH_2C_6H_5$

Il est préparé comme le composé (I) de l'exemple 1, à partir d'amino-2 phényl-3 propionate d'éthyle chlorhydrate (III, —A—B— = —CO—CH— ; R″ = $C_2H_5$) et d'éthoxy-2 phényl-4Δl-pyrroline (II,
|
$CH_2C_6H_5$

n = 1 ; $R_1$ = H ; $R_2$ = $C_6H_5$). Le produit intermédiaire (IV), obtenu sous forme de chlorhydrate par évaporation du solvant, est cristallisé dans l'isopropanol. $F_{inst}$ = 150 °C.

La cyclisation en produit (I) est effectuée par chauffage en solution éthanolique contenant HCl.

La base est libérée par alcalinisation, extraite au chloroforme et distillée sous pression réduite.

$eb_{0,2}$ = 208-214 °C. Rendt global : 34 %.

## Exemple 5

Composé (I) —A—B— = —CO—CH— ; n = 1 ; $R_1$ = H ; $R_2$ = $C_6H_5$.
|
$COOC_2H_5$

Il est préparé comme le composé (I) de l'exemple 1 à partir de chlorhydrate d'amino-malonate d'éthyle (III, —A—B— = —CO—CH— ; R″ = $C_2H_5$) et d'éthoxy-2 phényl-4Δl-pyrroline (II, n = 1,
|
$COOC_2H_5$

$R_1$ = H ; $R_2$ = $C_6H_5$). Après évaporation du solvant, le résidu est repris par du chloroforme, transformé en base par lavage avec une solution saturée de $NaHCO_3$ en excès, et séché. Le résidu d'évaporation du chloroforme est cristallisé par addition d'acétate d'éthyle. $F_{inst}$ = 215 °C (déc) - Rendt : 53 %.

## Exemple 6

Composé (I) —A—B— = —CO—CH— ; n = 1 ; $R_1$ = H ; $R_2$ = $C_6H_5$.
|
$CH_2COOC_2H_5$

Il est préparé comme le dérivé (I) de l'exemple 1 à partir d'amino-2 succinate d'éthyle, chlorhydrate (III, —A—B— = —CO—CH— ; R″ : $C_2H_5$) et d'éthoxy-2 phényl-4Δl-pyrroline. Sans isolement du
|
$CH_2COOC_2H_5$

dérivé IV, on obtient par évaporation du solvant et alcanisation la base cherchée, distillée sous pression réduite.

$eb_{0,5}$ = 220-224 °C - Rendt : 39 %.

## Exemple 7

Composé (I) —A—B— = —CO—CH— ; n = 1 ; $R_1$ = H ; $R_2$ = $C_6H_5$.
|
$(CH_2)_2COOC_2H_5$

Il est préparé comme le dérivé (I) de l'exemple 1 à partir du glutamate d'éthyle, chlorhydrate (III, —A—B— = —CO—CH— , R″ = $C_2H_5$ et d'éthoxy-2 phényl-4Δl-pyrroline.
|
$(CH_2)_2COOC_2H_5$

Base : $eb_{0,2}$ = 204-208 °C - Rendt : 28 %.

## Exemple 8

Composé (I) —A—B— = —CO—CH— ; n = 1 ; $R_1$ = H ; $R_2$ = $C_6H_5$.
|
$(CH_2)_2—SCH_3$

Il est préparé comme le dérivé (I) de l'exemple 1 à partir du chlorhydrate de l'ester éthylique de la méthionine, (III, —A—B— = —CO—CH— ; R″ = $C_2H_5$) et d'éthoxy-2 phényl-4ΔI-pyrroline.
(CH2)2—SCH3 [substituent on CH: $(CH_2)_2—SCH_3$]

Base : $eb_{0,3}$ = 208-213 °C - Rendt : 37 %.

## Exemple 9

Composé (I) —A—B— = —CO—CH— ($CH_2C_6H_5$) ; n = 1 ; $R_1 = CH_2C_6H_5$ ; $R_2$ = H.

Il est préparé comme le dérivé (I) de l'exemple 1 à partir d'amino-2 phényl-3 propionate d'éthyle chlorhydrate (III, —A—B— = —CO—CH— ($CH_2C_6H_5$) ; R″ = $C_2H_5$) et d'éthoxy-2 benzyl-5ΔI-pyrroline (II, n = 1 ; $R_1 = CH_2C_6H_5$ ; $R_2$ = H). Le produit intermédiaire (IV) est obtenu sous forme de base huileuse, après évaporation du solvant, alcalinisation et extraction à l'éther. Rendt : 86 %.

Le dérivé (IV) brut est cyclisé par chauffage sous pression réduite : le produit (I) formé est distillé. $eb_{0,1}$ = 200 °C - Rendt : 45 %.

## Exemple 10

Composé (I) —A—B— = 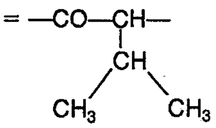 ; n = 2 ; $R_1$ = H ; $R_2 = C_6H_5$.

(chlorhydrate)

Il est préparé comme le dérivé (I) de l'exemple 1 à partir de DL-valine (III, —A—B— = —CO—CH— ($iC_3H_7$) ; R″ = H) et d'éthoxy-2 phényl-5 tétrahydro-3,4,5,6-pyridine (II, n = 2 ; $R_1$ = H ; $R_2 = C_6H_5$). La base obtenue par évaporation du solvant est transformée en chlorhydrate par une solution d'HCl dans l'isopropanol.

$F_{inst}$ = 191-193 °C - Rendt : 37 %.

## Exemple 11

Composé (I) —A—B— = —CO—CH— ($C_6H_5$) ; n = 2 ; $R_1$ = H ; $R_2 = C_6H_5$.

(chlorhydrate)

Il est préparé comme le dérivé (I) de l'exemple 1 à partir d'acide amino-2 phényl-2 acétique (III, —A—B— = —CO—CH— ($C_6H_5$) ; R″ = H) et d'éthoxy-2 phényl-5 tétrahydro-3,4,5,6-pyridine (II, n = 2 ; $R_1$ = H ; $R_2 = C_6H_5$). Après ébullition à reflux dans l'éthanol puis refroidissement, le dérivé intermédiaire (IV) est séparé par filtration et cyclisé par chauffage dans l'acide acétique à ébullition, pendant 8 h. L'évaporation de l'acide acétique laisse un résidu qui est repris par une solution éthanolique d'HCl, à chaud : le chlorhydrate du dérivé (I) cristallise à froid. $F_{inst}$ = 250-255 °C (déc) - Rendt : 30 %.

## Exemple 12

Composé (I) —A—B— = —CO—CH— ($COOC_2H_5$) ; n = 2 ; $R_1$ = H ; $R_2 = C_6H_5$.

(chlorhydrate)

Il est préparé comme le dérivé (I) de l'exemple 1 à partir de chlorhydrate d'aminomalonate d'éthyle (III, —A—B— = —CO—CH— ($COOC_2H_5$) ; R″ = $C_2H_5$) et d'éthoxy-2 phényl-5-tétrahydro-3,4,5,6-pyridine, (II, n = 2, $R_1$ = H ; $R_2 = C_6H_5$). Le chlorhydrate est cristallisé dans un mélange éthanol-oxyde d'isopropyle. $F_{inst}$ = 224 °C - Rendt : 42 %.

Exemple 13

Composé (I) —A—B— = —CO—CH— ; n = 2 ; $R_1$ = H ; $R_2$ = $C_6H_5$.
$(CH_2)_2COOC_2H_5$

(chlorhydrate)

Il est préparé comme le dérivé (I) de l'exemple 1 à partir de glutamate d'éthyle, chlorhydrate (III, —A—B— = —CO—CH— ($(CH_2)_2COOC_2H_5$) ; R″ = $C_2H_5$) et d'éthoxy-2 phényl-5-tétrahydro-3,4,5,6-pyridine, (II, n = 2 ; $R_1$ = H ; $R_2$ = $C_6H_5$). La base (I) ($eb_{0,05}$ = 205-215 °C) est transformée en chlorhydrate qui cristallise dans l'isopropanol. $F_{inst}$ = 168-170 °C.

Exemple 14

Composé (I) —A—B— = —CO—$CH_2CH_2$— ; n = 1 ; $R_1$ = H ; $R_2$ = $C_6H_5$.

Il est préparé comme le dérivé (I) de l'exemple 1 à partir d'acide amino-3 propionique (III, —A—B— = —CO—$CH_2$—$CH_2$— ; R″ = H) et d'éthoxy-2 phényl-4ΔI-pyrroline. Le produit intermédiaire (IV) R″ = H est obtenu par évaporation du solvant et cristallisation dans l'isopropanol. $F_{inst}$ = 215 °C - Rendt : 70 %.

Le dérivé (IV) est cyclisé par chauffage à 180-200 °C sous pression réduite, puis distillé ($eb_{0,5-1}$ = 180-210 °C) et cristallise dans l'éther isopropylique. $F_{inst}$ = 99 °C - Rendt : 72 %.

Exemple 15

Composé (I) —A—B— = —CO—$CH_2CH_2$— ; n = 1 ; $R_1$ = H ;

$R_2$ = 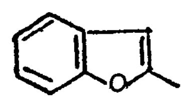

Il est préparé comme le dérivé (I) de l'exemple 1 à partir d'acide amino-3 propionique (III, —A—B— = —CO—$CH_2CH_2$— ; R″ = H) et d'éthoxy-2 (benzofuryl-2)-4ΔI-pyrroline. Après 20 h à reflux, l'éthanol est évaporé et le résidu distillé sous pression réduite. $eb_{0,4}$ = 202-208 °C — $F_{inst}$ : 120 °C - Rendt : 69 %.

Exemple 16

Composé (I) —A—B— = —CO—CH—$CH_2$— ; n = 1 ; $R_1$ = H ; $R_2$ = $C_6H_5$.
$C_6H_5$

Il est préparé comme le dérivé (I) de l'exemple 1 à partir de phényl-2 amino-3 propionate d'éthyle, chlorhydrate (III, —A—B— = CO—CH—$CH_2$ ($C_6H_5$) ; R″ = $C_2H_5$) et d'éthoxy-2 phényl-4ΔI-pyrroline.

La base est cristallisée dans l'acétate d'éthyle.
$F_{inst}$ = 164 °C - Rendt : 50 %.

Exemple 17

Composé (I) —A—B— = CO—$CH_2CH_2$— ; n = 1 ; $R_1$ = $C_6H_5$ ; $R_2$ = H.

Il est préparé comme le dérivé (I) de l'exemple 1 à partir d'acide amino-3 propionique (III, —A—B— = —CO-$CH_2$—$CH_2$— ; R″ = H) et d'éthoxy-2 phényl-5ΔI-pyrroline (II, n = 1 ; $R_1$ = $C_6H_5$ ; $R_2$ = H). Le produit intermédiaire (IV) est recristallisé dans le méthanol.
$F_{inst}$ = 178 °C - Rendt : 45 %.

Le dérivé (IV) est cyclisé par chauffage vers 200 °C sous pression réduite. Le produit (I) formé est distillé ($eb_{0,2-0,3}$ = 154-156 °C) - Rendt : 74 %.

Exemple 18

Composé (I) —A—B— = —CO—$CH_2CH_2CH_2$— ; n = 1 ; $R_1$ = H ; $R_2$ = $C_6H_5$.

Il est préparé comme le dérivé (I) de l'exemple 1 à partir d'acide amino-4 butyrique (III, —A—B— = —CO—$CH_2CH_2$—$CH_2$— ; R″ = H) et d'éthoxy-2 phényl-4ΔI-pyrroline. Après évaporation du solvant, le dérivé intermédiaire (IV) est cristallisé dans l'isopropanol.

$F_{inst}$ = 200 °C - Rendt : 73 %.

Le dérivé (IV) est cyclisé par chauffage à 200 °C sous pression réduite et le produit (I) formé est distillé.

$eb_{0,1}$ = 162-166 °C - Rendt : 11 %.

Les composés de formule I présentent des propriétés pharmacologiques intéressantes, particulièrement dans le domaine du système nerveux central, en tant qu'antidépresseurs non anticholinergiques. Leur toxicité n'apparaît qu'à des doses très supérieures aux doses pharmacologiquement actives, ce qui permet leur utilisation en thérapeutique pour le traitement des états dépressifs, sans les effets annexes gênants des antidépresseurs classiques.

On donnera ci-après des résultats des études toxicologiques et pharmacologiques qui mettent en évidence ces propriétés.

a) Toxicité aiguë chez la souris

Chaque composé a été administré par voie orale ou intrapéritonéale en une seule fois. Le comportement et la mortalité ont été observés pendant plusieurs heures après le traitement, puis quotidiennement pendant au moins une semaine.

Les résultats sont donnés dans le tableau ci-après.

b) Activité antidépressive

L'activité antidépressive de chaque composé a été évaluée chez la souris d'après sa capacité d'antagoniser la ptose palpébrale provoquée par une injection de réserpine. Les différents produits ont été administrés par voie orale en traitement préventif 1 heure avant l'injection intrapéritonéale de réserpine (2 mg/kg). L'intensité de la ptose palpébrale a été ensuite notée d'heure en heure pendant 3 heures, au moyen d'une cotation arbitraire semi-quantitative (cotation de Rubin). Les produits testés ont été administrés à plusieurs niveaux de doses et pour la plupart d'entre eux la dose efficace 50 % ($DE_{50}$) inhibant de 50 % la ptose palpébrale a pu être estimée.

Les résultats sont donnés dans le tableau ci-après.

| exemple n° | $DL_{50}$ po(mg/kg) | $DL_{50}$ ip(mg/kg) | ptose réserpine |
|---|---|---|---|
| 1 | $>200$ | $>200$ | $DE_{50} \simeq 33$mg/kg |
| 2 | $>200$ | $>200$ | $DE_{50} > 30$mg/kg |
| 3 | $>200$ | $>200$ | $DE_{50} \simeq 30$mg/kg |
| 4 | $>200$ | $<200$ | $DE_{50} < 30$mg/kg |
| 5 | $>200$ | $>200$ | $DE_{50} \simeq 7$mg/kg |
| 6 | $\gg 200$ | $\gg 200$ | $DE_{50} \simeq 100$mg/kg |
| 7 | $\gg 200$ | $\gg 200$ | $DE_{50} \simeq 30$mg/kg |
| 8 | $\gg 200$ | $\gg 200$ | $DE_{50} \simeq 30$mg/kg |
| 10 | $>200$ | $>200$ | $DE_{50} \simeq 100$mg/kg |
| 12 | $>200$ | $>200$ | $DE_{50} \simeq 30$mg/kg |

| exemple n° | $DL_{50}$ po(mg/kg) | $DL_{50}$ ip(mg/kg) | ptose réserpine |
|---|---|---|---|
| 13 | $> 200$ | $> 200$ | $DE_{50} \simeq 30 mg/kg$ |
| 14 | $\gg 200$ | $\gg 200$ | $DE_{50} = 5 mg/kg$ |
| 15 | $> 200$ | $> 200$ | $DE_{50} \simeq 33 mg/kg$ |
| 17 | $> 200$ | $> 200$ | $DE_{50} < 100 mg/kg$ |
| 18 | $> 200$ | $> 200$ | $DE_{50} \ll 33 mg/kg$ |

c) Activité atropinique

Les antidépresseurs classiques présentant un effet secondaire gênant de caractère atropinique, cette propriété éventuelle a été recherchée systématiquement pour les composés de formule I.

Les différents produits ont été administrés par voie orale 60 minutes avant l'injection sous-cutanée d'oxotrémorine à la dose de 2 mg/kg.

15 minutes, puis 30 et 45 minutes après l'injection de l'agent cholinergique, les différents symptômes reflétant une stimulation parasympathique ont été évalués selon une cotation arbitraire semi-quantitative (de 0 à 4) :

— symptômes de stimulation cholinergique périphérique : larmoiement, salivation ;

— symptômes de stimulation cholinergique centrale : tremblements, phénomène de Straub.

La quasi-totalité des composés étudiés dans ces conditions ne modifie aucunement les effets cholinergiques périphériques et centraux de l'oxotrémorine ; un seul composé présente une activité inhibitrice légère à la dose de 100 mg/kg (exemple n° 10).

d) Effets sur le système cardiovasculaire

Les composés de formule I ont été administrés chez le chien par voie intraveineuse, à des doses allant de 0,1 mg/kg à 10 mg/kg. Les variations de la pression artérielle, du débit artériel fémoral, de la résistance vasculaire, consécutives à ces injections, ont été enregistrées.

Les composés testés sont pour la plupart sans action sur la pression artérielle ; un seul d'entre eux est légèrement vasoconstricteur (exemple n° 5), deux autres étant légèrement hypotenseurs (exemples n° 6 et 8).

L'absence d'effets tensionnels pour la plupart de ces produits permet d'exclure une activité sympathomimétique chez les composés de formule I.

Les composés de formule I présentent un intérêt thérapeutique important dans le traitement des syndromes dépressifs et le ramollissement cérébral, et se montrent dénués des effets secondaires habituels gênants des produits appartenant à cette classe thérapeutique (effets atropiniques, effets tensionnels).

Ces composés sont administrables à l'homme par voie orale ou rectale à l'état de bases ou de sels (en comprimés, gélules, gouttes ou suppositoires) ou par voie parentérale sous forme de solution aqueuse d'un sel hydrosoluble ou sous une forme galénique assurant une résorption programmée.

Les différentes présentations peuvent contenir de 10 à 1 000 mg de principe actif par unité de prise pour l'administration par les voies orale et rectale, et de 1 à 500 mg de principe actif pour les autres voies d'administration.

La posologie journalière peut varier de 10 mg à 5 g selon les voies d'administration et les indications thérapeutiques envisagées.

**Revendications**

1. Composés de formule

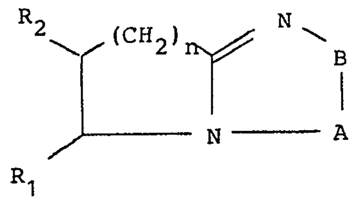

(I)

dans laquelle

*n* est égal à 1 ou 2,

$R_1$ représente un atome d'hydrogène et $R_2$ représente un groupe phényle ou un groupe benzofuryle-2, ou bien $R_2$ représente un atome d'hydrogène et $R_1$ représente un groupe phényle ou un groupe benzyle, et

le groupement —A—B— représente un groupe de formule

$$-CO-\underset{\displaystyle R}{\underset{|}{CH}}-,\quad -CO-\underset{\displaystyle R}{\underset{|}{CH}}-CH_2 \quad \text{ou} \quad -CO-(CH_2)_3-$$

R représentant un atome d'hydrogène, un radical alcoyle en $C_1$ à $C_6$, un groupe phényle, un groupe benzyle, un groupe méthylthioéthyle, ou un groupe de formule $-(CH_2)_m-COOR'$, *m* étant égal à 0, 1 ou 2 et R' étant un radical alcoyle en $C_1$ à $C_6$,

ainsi que leurs sels avec des acides pharmacologiquement acceptables.

2. Composé selon la revendication 1, caractérisé en ce que n = 1, $R_1$ est un atome d'hydrogène, $R_2$ est un groupe phényle et —A—B— est le groupe $-CO-CH_2-CH_2-$.

3. Composé selon la revendication 1, caractérisé en ce que n = 1, $R_1$ est un atome d'hydrogène, $R_2$ est un groupe phényle et —A—B— est un groupe $-CO-CH_2-CH_2-CH_2-$.

4. Composition thérapeutique ayant notamment un effet anti-dépresseur sur le système nerveux central, caractérisé en ce qu'elle comprend à titre de principe actif un composé tel que défini à l'une quelconque des revendications 1 à 3.

5. Procédé de préparation de composés de formule I telle que définie à la revendication 1, caractérisé en ce qu'on fait réagir un composé de formule

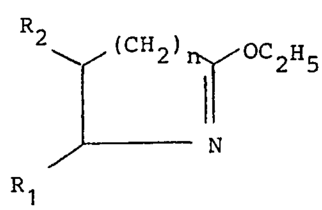

(II)

dans laquelle *n*, $R_1$ et $R_2$ ont la signification donnée à la revendication 1 avec une amine de formule

$$R''O-A-B-NH_2 \qquad \text{(III)}$$

dans laquelle A et B ont la signification donnée à la revendication 1 et R'' représente un atome d'hydrogène ou un radical alcoyle en $C_1$ à $C_6$, obtenant ainsi un composé de formule

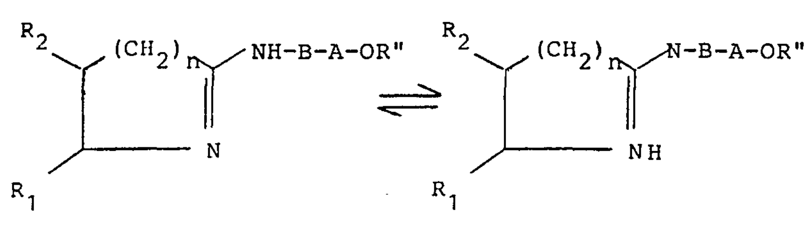

(IV)

et on cyclise le composé ainsi obtenu de formule IV en un composé de formule I.

6. Procédé selon la revendication 5, caractérisé en ce qu'on effectue la réaction du composé de formule II avec l'amine de formule III par chauffage à l'ébullition dans l'éthanol ou l'isopropanol.

7. Procédé selon la revendication 5, caractérisé en ce qu'on effectue la cyclisation par chauffage.

## Claims

1. Compounds having the formula :

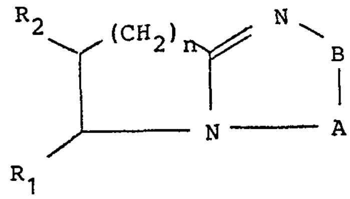

(I)

in which.
*n* is equal to 1 or 2,
$R_1$ represents a hydrogen atom and $R_2$ represents a phenyl group or a benzofuryl group ; or $R_2$ represents a hydrogen atom and $R_1$ represents a phenyl group or a benzyl group ; and grouping —A—B— represents a group of the formula :

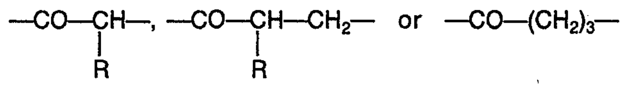

or

in which
R represents a hydrogen atom, a $C_{1-6}$ alkyl radical, a phenyl group, a benzyl group, a methylthioethyl group, or a group having the formula $-(CH_2)_m-COOR'$ in which *m* is equal to 0, 1 or 2 and R' is a $C_{1-6}$ alkyl radical,
and their pharmacologically acceptable acid addition salts.

2. Compound as claimed in claim 1, wherein $n = 1$, $R_1$ is a hydrogen atom, $R_2$ is a phenyl group and —A—B— is the group $-CO-CH_2-CH_2-$.

3. Compound as claimed in claim 1, wherein $n = 1$, $R_1$ is a hydrogen atom, $R_2$ is a phenyl group and —A—B— is a group $-CO-CH_2-CH_2-CH_2-$.

4. Therapeutic composition having in particular an antidepressant activity on the central nervous system, comprising, as active ingredient, a compound as defined in any one of claims 1-3.

5. Process for the preparation of compounds of the formula (I) as defined in claim 1, comprising reacting a compound of the formula :

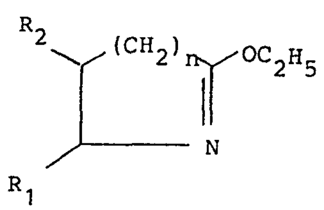

(II)

in which *n*, $R_1$ and $R_2$ are as defined in claim 1, with an amine having the formula :

$$R''O-A-B-NH_2 \qquad (III)$$

in which A and B are as defined in claim 1 and R'' represents a hydrogen atom or a $C_{1-6}$ alkyl radical, to give a compound of the formula :

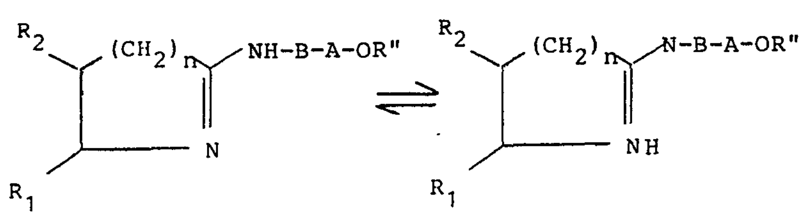

(IV)

and then cyclizing the resulting compound of the formula (IV) to give a compound of the formula (I).

6. Process as claimed in claim 5, wherein the reaction of the compound of the formula (II) with the amine of the formula (III) is effected by heating under boiling conditions within ethanol or isopropanol.

7. Process as claimed in claim 5, wherein the cyclization is effected by heating.

**Ansprüche**

1. Verbindungen der Formel

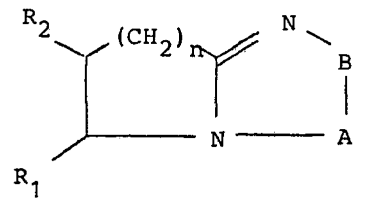

(I)

in der

*n* gleich 1 oder 2 ist,

$R_1$ ein Wasserstoffatom und $R_2$ ein Phenylrest oder ein Benzofuryl-2-rest ist oder $R_2$ ein Wasserstoffatom und $R_1$ ein Phenylrest oder ein Benzylrest ist und

die Gruppierung —A—B— ein Gruppe der Formel

$$-CO-\underset{R}{\underset{|}{CH}}-,\quad -CO-\underset{R}{\underset{|}{CH}}-CH_2 \quad \text{oder} \quad -CO-(CH_2)_3-$$

ist, worin

R ein Wasserstoffatom, ein $C_1$-$C_6$-Alkylrest, ein Phenylrest, ein Benzylrest, ein Methylthioethylrest oder eine Gruppe der Formel $-(CH_2)_m-COOR'$ ist, worin m gleich 0, 1 oder 2 und R' ein $C_1$-$C_6$-Alkylrest ist,

sowie ihre Salze mit pharmakologisch unbedenklichen Säuren.

2. Verbindung nach Anspruch 1, dadurch gekennzeichnet, daß n = 1, $R_1$ ein Wasserstoffatom, $R_2$ ein Phenylrest und —A—B— die Gruppe $-CO-CH_2-CH_2-$ ist.

3. Verbindung nach Anspruch 1, dadurch gekennzeichnet, daß n = 1, $R_1$ ein Wasserstoffatom, $R_2$ ein Phenylrest und —A—B— ein Gruppe $-CO-CH_2-CH_2-CH_2-$ ist.

4. Arzneimittelzubereitung mit insbesondere antidepressiver Wirkung auf das Zentralnervensystem, dadurch gekennzeichnet, daß sie als Wirkstoff eine Verbindung enthält, wie sie in einem beliebigen der Ansprüche 1 bis 3 definiert wurde.

5. Verfahren zur Herstellung von Verbindungen der Formel I, wie sie in Anspruch 1 definiert wurde, dadurch gekennzeichnet, daß man eine Verbindung der Formel

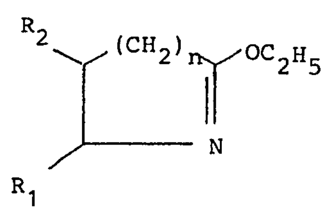

(II)

in der n, $R_1$ und $R_2$ die in Anspruch 1 genannten Bedeutungen haben, mit einem Amin der Formel

$$R''O-A-B-NH_2 \qquad \text{(III)}$$

in der A und B die in Anspruch 1 genannten Bedeutungen haben und R" ein Wasserstoffatom oder ein $C_1$-$C_6$-Alkylrest ist, umsetzt und hierdurch eine Verbindung der Formel

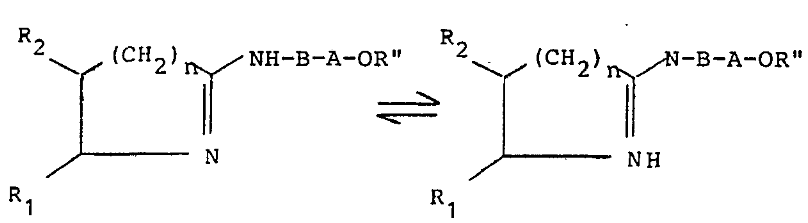

(IV)

bildet und die so erhaltene Verbindung der Formel IV zu einer Verbindung der Formel I cyclisiert.

6. Verfahren nach Anspruch 5, dadurch gekennzeichnet, daß man die Umsetzung der Verbindung der Formel II mit dem Amin der Formel III durch Erhitzen zum Sieden in Äthanol oder Isopropanol durchführt.

7. Verfahren nach Anspruch 5, dadurch gekennzeichnet, daß man die Cyclisierung durch Erhitzen durchführt.